# EUROPEAN PATENT APPLICATION

(11) **EP 1 872 753 A1**
(43) Date of publication of application: **02.01.2008**
(21) Application number: 07110595.1
(22) Date of filing: 19.06.2007
(51) Int. Cl.: A61F 9/008, A61B 18/20

(54) **Multifunction surgical probe**

(30) Priority: 30.06.2006 US 479667
(71) Applicant: Alcon, Inc., 6331 Hünenberg (CH)
(72) Inventor: Dacquay, Bruno, Irvine, TX 92603 (US); Horvath, Christopher, Irvine, CA 92604 (US); Romoda, Laszlo, San Clemente, CA 92673 (US); Pastrana, Jr., Armando, Forth Worth, TX 76109 (US)
(74) Representative: Hanna, Peter William Derek

(57) **Abstract**

A multifunction surgical probe (10,100) is disclosed, one embodiment comprising: a handpiece (12), operable to receive a light beam from a laser (28); and a firing switch (20) operable to provide a firing control signal to cause the laser to fire and emit the light beam. The surgical probe can further comprise an interface (16) communicatively coupled to the firing switch and operable to communicate the firing control signal to the laser. The laser can be operably coupled to a surgical console (26) operable to control the laser, and the interface can be communicatively coupled to the firing switch and operable to communicatively couple the surgical probe to the surgical console and to communicate the firing control signal to the surgical console. The surgical console (26) can be operable to control the laser (28) based on the firing control signal. The surgical probe can also comprise an interlock switch (24) operable to prevent the firing switch from firing the laser and a standby switch (22) operable to produce a standby control signal to switch the laser between a stand-by and a ready state. The firing switch (20) can be a progressively actuated switch (60) operable, at a first point (62) in its range of motion, to provide an initializing control signal operable to initialize the laser in preparation for firing and then, at a second point (64) in its range of motion, provide the firing control signal to cause the laser to fire.

## Description

### TECHNICAL FIELD OF THE INVENTION

The present invention relates generally to surgical probes, and more particularly, to a multifunction surgical probe operable to provide a surgeon independent control of an ophthalmic laser surgical system.

### BACKGROUND OF THE INVENTION

Handheld probes are used with a variety of electrical and mechanical equipment and, in particular, have become an accepted part of microsurgical and ophthalmic systems. Accordingly, the present handheld probe invention will be described in terms of its use with microsurgical systems and, in particular, its use with ophthalmic laser surgical systems.

When surgically treating a patient, for example, during ophthalmic surgery, a surgeon may use a complex patient treatment apparatus/surgical system that may require the control of a variety of different pneumatic and electronically driven subsystems. Typically, the operation of these subsystems is controlled by a microprocessor-driven console. The microprocessor within the surgical console may receive mechanical inputs from either the surgeon/operator or from an assistant to the surgeon/operator. For example, an assistant may directly manipulate controls on the surgical console, while the surgeon/operator may use a control input device, such as a footswitch, to provide mechanical inputs. In the case of a footswitch, the mechanical inputs originate from the movement of the surgeon's foot to control the operation of a subsystem within the surgical system. The mechanical inputs are translated into electrical signals that are then fed to the microprocessor to control the operational characteristics of the desired subsystem. One example of such a subsystem is a laser system used in ophthalmic laser eye surgery, such as the EYELITE^{®} photocoagulator manufactured by Alcon Laboratories, Inc. of Irvine, California.

Examples of footswitches designed for translating mechanical inputs into control signals for a complex patient treatment apparatus may be found in several U.S. Patents, including U.S. Patent Nos. 4,837,857 (Scheller, et al.), 4,965,417 (Massie), 4,983,901 (Lehmer), 5,091,656 (Gahn), 5,268,624 (Zanger), 5,554,894 (Sepielli), 5,580,347 (Reimels), 5,635,777 (Telymonde, et al), 5,787,760 (Thorlakson), 5,983,749 (Holtorf), and 6,179,829 B1 (Bisch, et al), and in International Patent Application Publication Nos. WO 98/08442 (Bisch, et al.), WO 00/12037 (Chen), and WO 02/01310 (Chen). These patents and patent applications focus primarily on footswitches that include a foot pedal or tiltable treadle similar to the accelerator pedal used to govern the speed of an automobile.

Certain footswitches, however, such as those used for ophthalmic laser surgery, may consist primarily of a casing having a switch operably connected to the laser surgical system. The switch is typically a single on/off type switch dedicated to firing a laser that has been previously placed in a "ready" condition. In a typical ophthalmic laser surgery, such as for photocoagulation, a laser subsystem is first prepared for the surgery by, for example, setting the proper parameters for the type of surgery, (e.g., power level, pulse duration, shot pattern, etc.) The laser is typically in a stand-by state (laser on, but incapable of being fired from the footswitch) when the laser system is powered on. For safety reasons, the user must take an affirmative step to place the laser in a "ready" condition from the stand-by state. A laser in the "ready" condition is configured to fire when the fire switch in the footswitch is actuated. Thus, there must be a conscious user decision to change the laser state from "stand-by" to "ready" by, for example, actuation of a button on the laser system console.

To place a laser in a "ready" condition, typically a surgical assistant must manipulate a control at the control panel of the surgical subsystem; for example, the assistant may press a button that switches the laser from stand-by to ready (and vice-versa). A surgical assistant typically performs this function because during a surgical procedure, at the point when the laser is needed, the surgeon is well into the surgery and not at a point where he/she may readily extricate from the surgical field to manipulate controls at the surgical console without disrupting the surgery. Further, in some instances the surgeon may be engaged in the surgical field and the assistant may also be otherwise engaged away from the control panel. A delay and inefficiency are imposed by requiring the assistant or the surgeon to move to the control panel to prepare the laser. A surgeon is thus typically dependent on a surgical assistant to perform this function for him or her.

Prior art systems do not provide a means by which a surgeon can, independently of an assistant, easily place a laser in a ready position and continue with the laser surgery without having to detract attention from the surgery. Instead, the surgeon must rely on an assistant. Once the laser is in a ready state, however, prior art systems do provide a firing switch in the laser system footswitch that the surgeon can actuate to fire the laser. Pending co-owned U.S. Patent Application No. (not yet known) entitled "Multifunction Surgical Switch", filed on June 26, 2006, teaches a multifunction surgical footswitch that can provide a surgeon the ability to independently transition a laser in an ophthalmic laser surgical system between a "stand-by" state and a "ready" state while maintaining his/her attention within a surgical field. However, even the footswitch disclosed in that application does not provide a surgeon the increased precision and synchronous control of a surgical laser that can be provided by a handheld probe having the ability to control the laser using controls incorporated into the handheld probe.

Therefore, a need exists for a multifunction surgical probe that can provide a surgeon the ability to independently operate a laser of an ophthalmic laser surgical system while maintaining his/her attention within a surgical field.

### BRIEF SUMMARY OF THE INVENTION

The present invention provides a multifunction surgical probe in accordance with claims which follow. Embodiments of the present invention can comprise a switching mechanism within a probe assembly that duplicates or replaces the prior art laser firing functionality of a surgical footswitch and, in some embodiments, the stand-by-to-ready laser state switching functionality of the controls on a main surgical console. One embodiment of the multifunction surgical probe of this invention is a surgical probe comprising: a handpiece, operable to receive a light beam from a laser; and a firing switch operable to provide a firing control signal to cause the laser to fire and emit the light beam. The surgical probe can further comprise an interface communicatively coupled to the firing switch and operable to communicate the firing control signal to the laser. The laser can be operably coupled to a surgical console operable to control the laser, and the interface can be operable to communicatively couple the surgical probe to the surgical console and to communicate the firing control signal to the surgical console. The surgical console can be operable to control the laser based on the firing control signal.

The surgical probe can also comprise an interlock switch operable to prevent the firing switch from firing the laser and a standby switch operable to produce a standby control signal to switch the laser between a stand-by and a ready state. The firing switch can be a progressively actuated switch operable, at a first point in its range of motion, to provide an initializing control signal operable to initialize the laser in preparation for firing and then, at a second point in its range of motion, provide the firing control signal to cause the laser to fire.

The firing switch, ready switch and interlock switch can be attached to the handpiece in any conventional manner as will be apparent to those having skill in the art. The switches can be positioned such that they can be activated by a middle finger, an index finger, a thumb, or any other part of a user's hand. The switches can comprise buttons, slide switches, spring loaded switches, touch-and-release, touch-and-hold, or any other conventional type switch as will be known to those having skill in the art. The switches can also be recessed, protected, or shielded to prevent unintentional operation. The interlock switch can be a mechanical interlock or can be operable to produce an interlock control signal to prevent or allow firing of the laser by the second switch.

Other embodiments of the surgical probe of this invention can comprise combinations of the above-described switches. For example, the surgical probe can comprise a firing switch and a ready switch, but no interlock switch. Or the probe can comprise a firing switch to fire the laser, but not a ready switch or an interlock switch. Any such combination of switches is contemplated to be within the scope of this invention. For example, the surgical probe can comprise only a ready switch operable to switch a laser between a stand-by and a ready state that can be used in combination with a typical prior art footswitch to fire the laser. Further, the ready switch and/or the interlock switch can be located on the surgical console. The interface can be an electrical cable coupling the switches to the surgical console, which can perform the signal processing, or a wireless interface that transmits control signals to the surgical console.

Embodiments of the surgical probe of this invention can also comprise a lift sensor assembly operable to detect lifting of the surgical probe from a surface and generate a control signal in response to the detected lifting. The control signal can be transmitted to the surgical console where it can be operable, for example, to cause the laser to warm-up in preparation for firing. The lift sensor assembly can be an ultrasound proximity sensor, an optical sensor, a radio frequency signal modulation sensor, a radar sensor, an accelerometer, or any other such sensor known to those having skill in the art and operable to detect the lifting of the surgical probe. Embodiments of the present invention can comprise a wired or wireless interface to communicate control signals between the surgical probe and the surgical console, and can include additional switches coupled to the handpiece and operable to produce additional control signals operable to control one or more additional functions at the surgical console. Embodiments of the present invention further include a surgical probe having a progressively actuated switch/laser-firing sequence and a method for firing a laser and/or switching a laser between a stand-by and a ready condition in accordance with the teachings of the present invention.

Embodiments of the present invention can be implemented within any ophthalmic surgical system as known to those in the art, such as the EYELITE^{®} Laser Surgical System manufactured by Alcon Manufacturing, Ltd. of Irvine, California. The embodiments of this invention can be incorporated within any such surgical machine or system for use in ophthalmic or other surgery. Other uses for a multifunction surgical probe designed in accordance with the teachings of this invention will be known to those having skill in the art and are contemplated to be within the scope of this invention.

### BRIEF DESCRIPTION OF THE SEVERAL VIEWS OF THE DRAWINGS

A more complete understanding of the present invention and the advantages thereof may be acquired by referring to the following description, taken in conjunction with the accompanying drawings in which like reference numerals indicate like features and wherein:
FIGURE 1 illustrates an embodiment of a surgical probe according to the present invention;
FIGURES 2 and 3 are views of the surgical probe of FIGURE 1 from varying angles to more clearly show switch locations;
FIGURE 4 is a functional diagram of an embodiment of the multifunction surgical probe of this invention having a wireless interface; and
FIGURE 5 is a functional diagram of an embodiment of the multifunction surgical probe of this invention having a progressive laser firing sequence.

### DETAILED DESCRIPTION OF THE INVENTION

Preferred embodiments of the present invention are illustrated in the FIGURES, like numerals being used to refer to like and corresponding parts of the various drawings.

The various embodiments of the present invention provide a multifunction surgical probe that allows a surgeon to place a surgical laser in a ready condition, fire the laser once it is in the ready condition, or both from the probe handpiece. Embodiments can comprise one or more switches for controlling various functions.

In the prior art, surgical lasers, such as an endolaser, are typically fired by a surgeon using a footswitch. The surgeon uses a surgical probe for receiving and directing a laser beam of light from the surgical laser into a patient's eye, but the laser itself is typically controlled via the footswitch and controls on a surgical console (that typically houses the surgical laser). The probe, with which the surgeon is directing the application of the laser beam, is thus physically displaced from the laser firing mechanism (footswitch). Further, during a laser surgery, a surgeon is routinely required to position himself or herself in various different positions relative to a patient's eye, requiring the surgeon to move around the patient. Consequently, the footswitch for firing the laser must also be routinely moved around during a surgery. This can result in a loss of efficiency, precision and control due to the physical separation between the laser delivery site and the firing switch. The embodiments of the present invention can provide increased precision and synchronous control with the laser location and firing by providing a means to operate the laser from the surgical probe instead of from a footswitch

Embodiments of the present invention can further provide increased functionality, such as warming up a laser prior to use, by means of a sensors, such as a lifting detector assembly, in the surgical probe handpiece to detect lifting of the probe from a surface. The lifting detector can generate a control signal in response to the detected lifting. The control signal can be transmitted to the surgical console where it can be operable to cause the laser to warm-up in preparation for firing. In this way, laser reliability can be increased while also decreasing lag times during the surgery. The lift sensor assembly can be selected from the group of sensors consisting of an ultrasound proximity sensor, an optical sensor, a radio frequency signal modulation sensor, a radar sensor, and an accelerometer, or any other such sensor known to those having skill in the art and operable to detect the lifting of the surgical probe.

Embodiments of the present invention can thus reduce a surgeon's dependence on his/her assistant during laser eye surgery, make the surgery flow more efficient and increase the surgeon's precision and control by allowing the surgeon to independently control the stand-by/ready condition of the laser, and/or fire the laser, from a single multifunction surgical probe. A dedicated mode switch on the laser console can be retained for redundancy, but need not be the only means for making the laser ready. The surgeon will therefore not need to use his or her feet or rely on an assistant to fire the laser or transition the laser from stand-by to ready, and vice versa, during a surgery, freeing the surgeon to dedicate his or her attention to the surgical field.

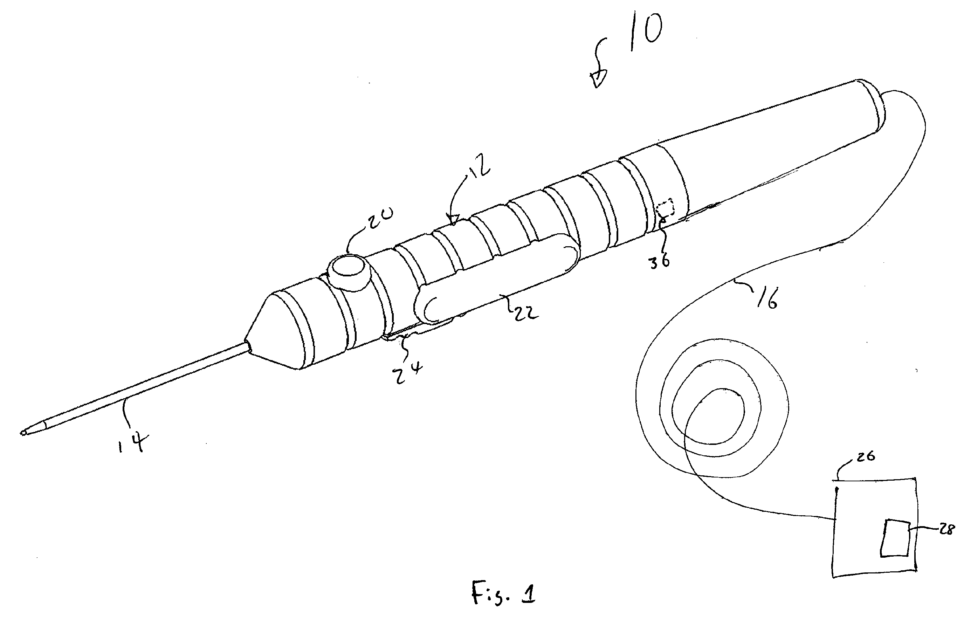

FIGURE 1 shows one embodiment of a surgical probe 10 according to this invention. Surgical probe 10 includes a handpiece (housing) 12 that comprises, in this embodiment, a ribbed tubular body. Probe 10 further comprises a probe tip 14, a laser firing switch 20, laser stand-by/ready switch 22 and interlock switch 24 all attached to or integral to handpiece 12. All of these components can be made from any suitable material, such as stainless steel, titanium, or plastic. With the exception of the novel control functions provided by switches 20, 22 and 24, and the control signals they generate and provide via interface 16 (described in greater detail below), probe 10 can be any generally known surgical laser probe, such as an endolaser probe, as will be apparent to those having skill in the art.

Laser firing switch 20, laser stand-by/ready switch 22, interlock switch 24 and any other switch that may be attached to handpiece 12 in the various embodiments of this invention can be attached to the handpiece 12 in any conventional manner as will be apparent to those having skill in the art. Laser firing switch 20 can be positioned so that it can readily and comfortably be activated by, for example, the index finger, middle finger or thumb of a typical user's hand. Laser stand-by/ready switch 22 and interlock switch 24 (and laser firing switch 20) can be positioned in any manner such that they will be easy to reach and operate by a user. Either of these switches can be recessed, protected or shielded to prevent unintentional firing of the laser 28, such as if the probe is dropped. The switches can comprise buttons, slide switches, spring loaded switches, touch-and-release, touch-and-hold, or any other conventional type switch as will be known to those familiar with the art.

Laser firing switch 20 is typically a press and hold type switch that can fire a single shot of varying duration or multiple shots, depending on the surgeon's configurable laser 28 settings. Laser stand-by/ready switch 22 is typically a single action button switch that can switch the laser mode from stand-by to ready (or vice-versa) upon pressing and release. Interlock switch 24 can be a mechanical interlock that physically prevents actuation of firing switch 20, for example, or can be a switch operable to produce and provide a control signal to the surgical console 26 to prevent or allow firing of the laser 28 by laser firing switch 20. Surgical console 26 includes for purposes of this invention, the control and/or processing circuitry for laser 28, whether surgical console 26 is a separate enclosure or the same enclosure as that of laser 28. Surgical console 26 can be any console housing laser 28, including, for example, a multi-purpose console, such as a vitreo-retinal surgical console that includes laser 28, or a dedicated laser 28 enclosure.

Interface 16 of surgical probe 10 can comprise one or more cable assemblies, to operably couple the surgical probe 10 to surgical console 26 and provide control signals from surgical probe 10 to surgical console 26. Surgical console 26 is operable to control laser 28, for example to cause laser 28 to switch modes and/or to fire based on the control signals that are relayed from the surgical probe 10 to the surgical console 26. Interface 16 can comprise an optical fiber for transmitting a laser light beam from laser 28, and a cable, such as a cable capable of carrying electronic signals, for relaying control signals between probe 10 and surgical console 26. Interface 16 serves as an interface, communicatively coupled to one or more of laser firing switch 20, laser stand-by/ready switch 22 and interlock switch 24, and operable to communicatively couple the surgical probe 10 to surgical console 26 to communicate control signals from the switches to the surgical console 26. Any suitable optical fiber and electronic signal cable, as will be known to those having skill in the art can be used within interface 16.

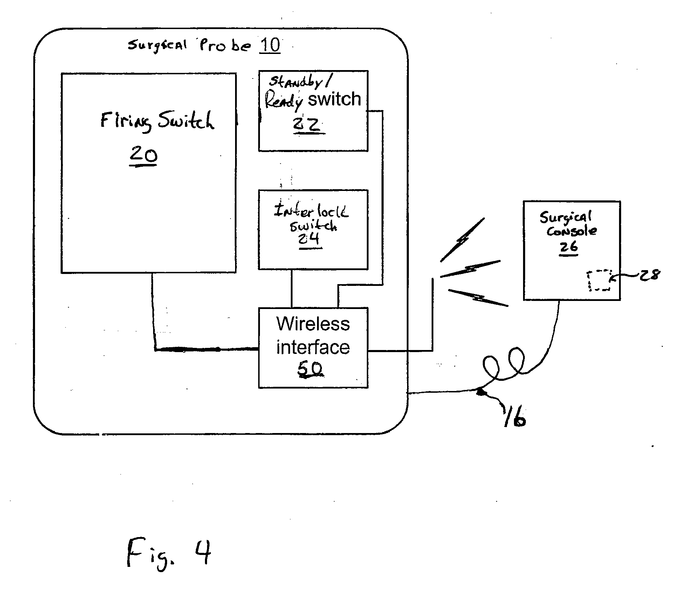

Other embodiments of surgical probe 10 can comprise a wireless interface 50, as shown in FIGURE 4, that is operable to establish a wireless communication pathway between surgical probe 10 and surgical console 26/laser 28 to accomplish the same control signal transmission described above in a wireless manner. Surgical console 26 and laser 28 can be, for example, an EYELITE^{®} photocoagulator manufactured by Alcon Laboratories, Inc. of Irvine, California.

The embodiment of FIGURE 1 shows laser stand-by/ready switch 22 and interlock switch 24 attached to handpiece 12. However, stand-by/ready switch 22 and/or interlock switch 24 can also be positioned, for example, on surgical console 26 in some embodiments. The position of stand-by ready switch 22 and interlock switch 24 can be changed to accommodate a given implementation, depending on the functionality desired to be implemented in surgical probe 10. Any combination of laser firing switch 20, laser stand-by/ready switch 22 and interlock switch 24 can be implemented on surgical probe 10. Further, embodiments of the surgical probe 10 can comprise one or more additional switches attached to handpiece 12 and each operable to produce a control signal operable to control a function at surgical console 26 (e.g., adjust laser power, pulse duration, etc.).

Embodiments of the surgical probe of this invention can also comprise sensors, such as lift sensor assembly 36, operable to detect lifting of the surgical probe 10 from a surface and to generate a control signal in response to the detected lifting. The control signal can be transmitted to the surgical console 26 where it can be operable, for example, to cause laser 28 to warm-up in preparation for firing, or otherwise prepare the laser surgical system for firing. The lift sensor assembly 36 can comprise, for example, an ultrasound proximity sensor, an optical sensor, a radio frequency signal modulation sensor, a radar sensor, and an accelerometer, or any other such sensor operable to sense movement, as known to those having skill in the art, and operable to detect the lifting of the surgical probe 10. Embodiments of the surgical probe 10 of this invention can comprise any combination of such sensors.

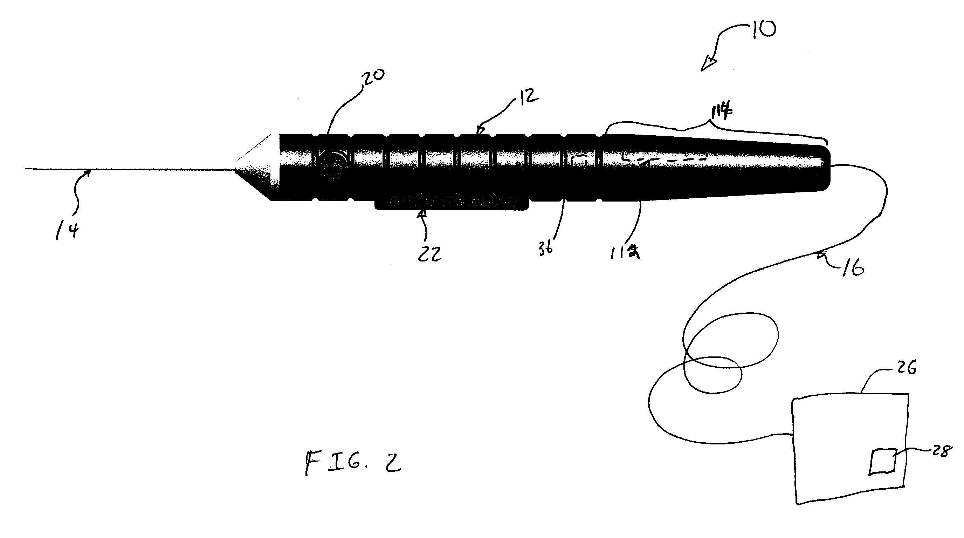

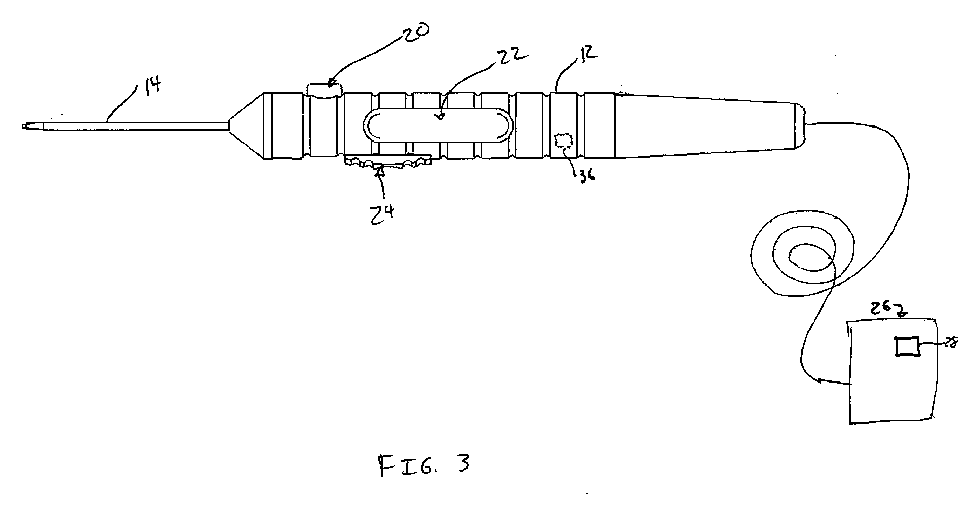

FIGURES 2 and 3 show an embodiment of surgical probe 10 of this invention from different perspectives to illustrate possible positions for the various switches. Interlock switch 24 can be a physical or electronic interlock, but in either case serves the purpose of a fail-safe and must be actuated before laser firing switch 20 can be functional to fire laser 28. Interlock switch 24 can be, for example, a slide switch that can be actuated and locked in a position to allow firing of laser 28 by laser firing switch 20 (FIGUREs 1 and 3). Interlock switch 24 can also be, in some embodiments, such as shown in FIGURE 2, a resistive/electrical sensor 112 in the proximal end 114, for example, of handpiece 12 that is actuated by contact with the user's hand, such as when it is positioned in the "v" formed at the base of the thumb and index finger during use of surgical probe 10. Such a resistive/electrical sensor 112 could be positioned such that it can be actuated by contact anywhere on proximal end 114, or any selected portion of handpiece 12. A resistive/electrical sensor switch of this type, as those having skill in the art will appreciate, comprises a circuit that is closed/completed by the user's hand. Such an interlock switch 22 can prevent accidental firing of the laser, for example, if the surgical probe 10 is dropped or set down on a surface.

FIGURE 4 is a functional diagram of an embodiment of the multifunction surgical probe of this invention incorporating a wireless interface 50 for communicating the control signals provided by the various switches of the present invention. Wired interface 16 in this embodiment can comprise an optical fiber for transmitting a laser light beam from laser 28 to the surgical probe 10 for introduction into a surgical site and not include electronic cable for communicating control signals to surgical console 26. In this embodiment, interlock switch 24 is an electrical interlock controlling the operation of laser firing switch 20. Wireless interface 50 can include an encoder for encoding of the control signals to be transmitted to surgical console 26.

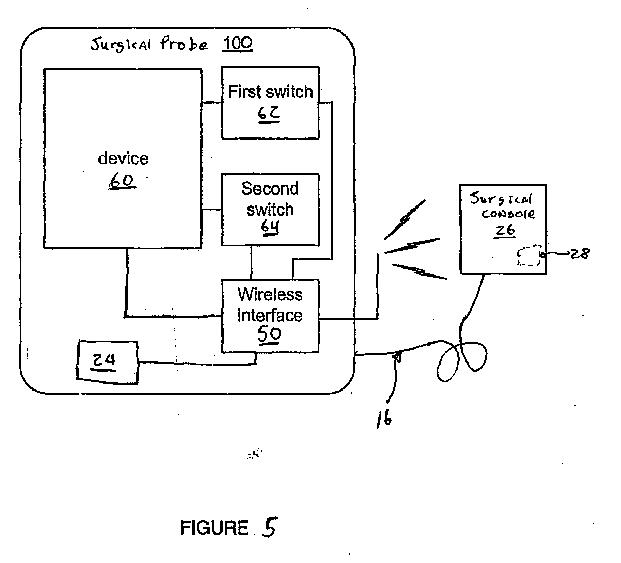

FIGURE 5 is a functional diagram of an embodiment of a surgical probe 100 of this invention having a progressive laser firing sequence. Although FIGURE 5 shows a wireless progressive laser firing sequence embodiment of this invention, a wired progressive laser firing embodiment is also contemplated to be within the scope of this invention, such embodiment having a wired interface 16 such as described with reference to FIGURE 1. This embodiment comprises an input device 60, analogous to the laser firing switch 20 of FIGURE 1, but having a progressive actuation functionality as described below. As in the other embodiments of the present invention, the embodiment of FIGURE 5 can include a combination of switches and functions as described herein, although it is shown in FIGURE 5 having only an interlock switch 24 and a mechanical input device 60 for firing laser 28. Surgical probe 100 includes two switches, first switch 62 and second switch 64, that operably couple to the input device 60. Input device 60 can be a switch, such as a push-button switch or slide switch, or any other such switch that can provide a progressive action as described herein and as will be familiar to those having skill in the art.

In operation, first switch 62 is actuated and generates a first control signal as input device 60 orients past a first determined point. The first control signal is operable, for example, to initialize surgical laser 28 within the surgical system 26. The first switch 62 may be actuated, for example, when the input device 60 is initially depressed. The second switch 64 produces a second control signal offset in time from the first control signal produced by the activation of first switch 62. For example, second switch 64 may be actuated as input device 60 nears the end of its range of motion (e.g., when a button switch is fully depressed). The second control signal may direct the firing of surgical laser 28.

The trigger time between the activation of first switch 62 and second switch 64 may allow, for example, the stress on the laser 28 to be reduced. In such an implementation, the trigger time between the activation of the first switch 62 and the second switch 64 allows the laser 28 to slowly warm up before firing. Note that the functionality of first switch 62 and second switch 64 can be incorporated within a single laser firing switch 20, such as described with reference to FIGURE 1. For example, referring back to FIGURE 1, stand-by/ready switch 22 may be actuated to place the laser 28 in a ready condition from a stand-by condition and interlock switch 24 actuated to allow firing of laser 28 from laser firing switch 20. Then, laser firing switch 20, which can incorporate in one embodiment the functions of first switch 62 and second switch 64, ramps the laser 28 up to firing and then fires the laser 28 in the continuous movement of the surgeon's finger from initially depressing laser firing switch 20/input device 60 (actuating first switch 62) to fully depressing laser firing switch 20/input device 60 (actuating second switch 64).

In such an embodiment, laser firing switch 20/input device 60 can comprise a progressively actuated push-button or slide-switch, for example, operably coupled to a multi-position switch or switches having the functionality of first switch 62 and second switch 64. In one embodiment, the trigger time between the activation of the two switches 62 and 64 can be between about 100 ms and 300 ms. The actual time may depend on the hand speed of the operator. This allows laser 28 to be slowly ramped to power over the span of about 100 ms to about 300 ms (note that this is after the laser has already been placed in a ready condition from a stand-by condition). This is particularly useful for certain lasers, known to those having skill in the art, that can not be turned on in less than 50 ms. The reduced stress associated with firing the laser in accordance with this embodiment of the present invention will result in improved laser performance and reliability. Although surgical probe 100 is illustrated in the embodiment of FIGURE 5 as establishing a wireless control signal communication pathway between the surgical probe 100 and surgical laser 28 (surgical console 26), surgical probe 100 may instead be physically coupled to the control circuits associated with initializing and firing laser 28, such as in the embodiment of FIGURE 1.

The present invention has been described by reference to certain preferred embodiments; however, it should be understood that it may be embodied in other specific forms or variations thereof without departing from its spirit or essential characteristics. The embodiments described above are therefore considered to be illustrative in all respects and not restrictive, the scope of the invention being indicated by the appended claims. As may be used herein, the terms "substantially" and "approximately" provide an industry-accepted tolerance for their corresponding term and/or relativity between items. Such an industry-accepted tolerance ranges from less than one percent to fifty percent and corresponds to, but is not limited to, component values, integrated circuit process variations, temperature variations, rise and fall times, and/or thermal noise. Such relativity between items ranges from a difference of a few percent to magnitude differences. As may also be used herein, the term(s) "coupled to" and/or "coupling" include direct coupling between items and/or indirect coupling between items via an intervening item (e.g., an item includes, but is not limited to, a component, an element, a circuit, and/or a module) where, for indirect coupling, the intervening item does not modify the information of a signal but may adjust its current level, voltage level, and/or power level. As may further be used herein, inferred coupling (i.e., where one element is coupled to another element by inference) includes direct and indirect coupling between two items in the same manner as "coupled to". As may even further be used herein, the term "operable to" indicates that an item includes one or more of power connections, input(s), output(s), etc., to perform one or more its corresponding functions and may further include inferred coupling to one or more other items. As may still further be used herein, the term "associated with", includes direct and/or indirect coupling of separate items and/or one item being embedded within another item. As may be used herein, the term "compares favorably", indicates that a comparison between two or more items, signals, etc., provides a desired relationship. For example, when the desired relationship is that signal 1 has a greater magnitude than signal 2, a favorable comparison may be achieved when the magnitude of signal 1 is greater than that of signal 2 or when the magnitude of signal 2 is less than that of signal 1.

While the present invention has been described with reference to the general area of laser ophthalmic surgery, the teachings contained herein can apply equally to any surgical system where it is desirous to control a laser subsystem.

## Claims

1. A surgical probe (10,100), comprising:
a handpiece (12), operable to receive a light beam from a laser (28); and
a firing switch (20,60,62,64) operable to provide a firing control signal to cause the laser to fire and emit the light beam.

2. The surgical probe of Claim 1, further comprising an interface (16) communicatively coupled to the firing switch (20,60,62,64) and operable to communicate the firing control signal to the laser (28).

3. The surgical probe of Claim 2, wherein the laser (28) is adapted to be operably coupled to a surgical console (26) operable to control the laser, and wherein the interface (16) is operable to communicatively couple the surgical probe (10) to the surgical console and to communicate the firing control signal to the surgical console, wherein the surgical console is operable to control the laser based on the firing control signal.

4. The surgical probe of Claim 2 or Claim 3, wherein the interface (16) is a wired interface comprising a cable for communicating the firing control signal to the laser (28) or the surgical console (26).

5. The surgical probe of Claim 2 or Claim 3, wherein the interface (16) is a wireless interface (50).

6. The surgical probe of Claim 2 or Claim 3, wherein the interface (16) further comprises an optical fiber operable to receive and transmit the light beam from the laser to the handpiece for delivery to a surgical site.

7. The surgical probe of any of Claims 1 to 3, further comprising an interlock switch (24) operable to prevent the firing switch (20) from firing the laser (28).

8. The surgical probe of Claim 7, wherein the interlock switch (24) is a mechanical interlock that mechanically prevents the operation of the firing switch (20).

9. The surgical probe of Claim 7, wherein the interlock switch (24) is operable to produce a second control signal to control firing of the laser (28) by actuation of the firing switch (20).

10. The surgical probe of Claim 1, further comprising a standby switch (22) operable to produce a standby control signal to switch the laser between a stand-by and a ready state.

11. The surgical probe of any of Claims 1 to 10, wherein the firing switch (20) is selected from the group consisting of a button switch, a slide switch, a spring-loaded switch, a touch and release switch, a press and hold switch, an a single-action switch.

12. The surgical probe of Claim 1, wherein the firing switch (20) is a press and hold switch.

13. The surgical probe of any of Claims 1 to 3, wherein operation of the firing switch (20) is adapted to be disabled by an interlock switch (24) at the laser (28) or the surgical console (26).

14. The surgical probe of Claim 3, wherein an interlock switch (24) is operable to produce an interlock control signal to allow firing of the laser (28) by actuation of the firing switch (20), and wherein the interface (16) is communicatively coupled to the firing switch and to the interlock switch and is operable to communicate the firing control signal and the interlock control signal to the surgical console (26), wherein the surgical console is operable to control the laser (28) based on the firing control signal and the interlock control signal.

15. The surgical probe of Claim 3, further comprising a ready switch (22) operable to produce a ready control signal to switch the laser (28) between a stand-by and a ready state, and wherein the interface (16) is communicatively coupled to the firing switch (20) and to the ready switch and operable to communicate the firing control signal and the ready control signal to the surgical console (26), wherein the surgical console is operable to control the laser based on the firing control signal and the ready control signal.

16. The surgical probe of any of Claims 1 to 15, further comprising a lift sensor assembly (36) operable to detect lifting of the surgical probe (10) from a surface and generate a lifting control signal in response to the detected lifting.

17. The surgical probe of Claim 16, when dependent on Claim 3, wherein the lift sensor assembly (36) is operable to detect lifting of the surgical probe (10) from a surface and generate a lifting control signal in response to the detected lifting, and wherein the interface is communicatively coupled to the lift sensor assembly and operable to communicate the lifting control signal to the surgical console, wherein the surgical console is operable to control the laser based on the lifting control signal.

18. The surgical probe of Claim 16 or Claim 17, wherein the lift sensor assembly (36) is selected from the group consisting of an ultrasound proximity sensor, an optical sensor, a radio frequency signal modulation sensor, a radar sensor, and an accelerometer.

19. The surgical probe of Claim 16 or Claim 17, wherein the lifting control signal is communicated to the laser (28) and is operable to cause the laser to warm-up in preparation for firing.

20. The surgical probe of Claim 1, further comprising one or more additional switches operable to produce one or more additional control signals operable to control one or more laser functions.

21. The surgical probe of Claim 20, wherein the one or more functions comprise laser power and pulse duration.

22. The surgical probe of any of Claims 1 to 21, wherein the firing switch (60,62,64) is a progressively actuated switch operable to, at a first point (62) in its range of motion, provide an initializing control signal operable to initialize the laser (28) in preparation for firing and then, at a second point (64) in its range of motion, provide the firing control signal to cause the laser to fire.

23. The surgical probe of any of Claims 1 to 22, wherein the surgical probe (10) is an endolaser probe.

24. The surgical probe of any of Claims 1 to 22, wherein the laser (28) is an ophthalmic surgical laser.

25. An ophthalmic surgical system, comprising:
a laser (28); and
a surgical probe (10,100), wherein the surgical probe comprises:
a handpiece (12), operable to receive a light beam from the laser; and
a switch (20,60,62,64) operable to provide a control signal to cause the laser to fire and emit the light beam.

26. The ophthalmic surgical system of Claim 25, further comprising:
a surgical console (26); and
an interface (16,50) communicatively coupled to the switch and operable to communicatively couple the surgical probe (10,100) to the surgical console (26) and to communicate the control signal to the surgical console, wherein the surgical console is operable to control the laser (28) based on the control signal.
